# EUROPÄISCHE PATENTSCHRIFT

(11) **EP 4 297 643 B1**
(45) Veröffentlichungstag und Bekanntmachung des Hinweises auf die Patenterteilung: **29.10.2025**
(21) Anmeldenummer: 22708882.0
(22) Anmeldetag: 21.02.2022
(51) Int. Cl.: A61B 5/06

(54) **MEDIZINISCHES SYSTEM ZUR KONTROLLE EINER POSITION UND EINER ORIENTIERUNG EINER KATHETERSPITZE IM KÖRPER EINES PATIENTEN**
MEDICAL SYSTEM FOR CONTROLLING THE POSITION AND ORIENTATION OF A CATHETER TIP IN A PATIENT'S BODY
SYSTÈME MÉDICAL POUR CONTRÔLER LA POSITION ET L'ORIENTATION DE LA POINTE D'UN CATHÉTER DANS LE CORPS D'UN PATIENT

(30) Priorität: 23.02.2021 DE 102021201700
(43) Veröffentlichungstag der Anmeldung: 03.01.2024
(73) Patentinhaber: B. Braun Melsungen AG, 34212 Melsungen (DE)
(72) Erfinder: WILDHAGEN, Jens, 30659 Hannover (DE); THALMANN, David, 34260 Kaufungen (DE); VUKOVIC, Dejana, 34212 Melsungen (DE)
(74) Vertreter: Patentanwälte Ruff, Wilhelm, Beier, Dauster & Partner mbB
(86) Internationale Anmeldenummer: PCT/EP2022/054220
(87) Internationale Veröffentlichungsnummer: WO 2022/179976

(56) Entgegenhaltungen:
- US-A1- 2008 097 232
- US-A1- 2018 132 877
- US-A1- 2019 038 897
- SCHUMMER W ET AL: "Modified ECG-guidance for optimal central venous catheter tip positioning; A transesophageal echocardiography controlled study", DER ANAESTHESIST ; ZEITSCHRIFT FÜR ANÄSTHESIE, INTENSIVMEDIZIN, NOTFALLUND KATASTROPHENMEDIZIN, SCHMERZMEDIZIN, SPRINGER, BERLIN, DE, vol. 54, no. 10, 1 October 2005 (2005-10-01), pages 983 - 990, XP019318739, ISSN: 1432-055X, DOI: 10.1007/S00101-005-0886-2

## Beschreibung

Die Erfindung betrifft ein medizinisches System zur Kontrolle einer Position und einer Orientierung einer Katheterspitze eines Katheters, insbesondere eines Zentralvenenkatheters, im Körper eines Patienten.

Katheter, insbesondere Zentralvenenkatheter, sind in der Medizin allgemein bekannt. Beim Anlegen eines Katheters wird dessen Katheterspitze in einen körperseitigen Zugang eingeführt und bis zu einem gewünschten Lageort vorgeschoben.

Zentralvenenkatheter werden in der Regel über eine Vene der oberen Körperhälfte in das Venensystem eingeführt. Die Katheterspitze wird üblicherweise bis in den Bereich des rechten Vorhofs vorgeschoben. Eine nicht ausreichend genaue Positionierung der Katheterspitze kann zu Problemen führen. Um dem entgegenzuwirken, ist eine Kontrolle der Position der Katheterspitze erforderlich. Hierfür sind in der klinischen Praxis unterschiedliche Verfahren bekannt.

Bei einem bekannten Verfahren wird nach oder bereits während des Anlegens die Position der Katheterspitze röntgenologisch erfasst. Die damit einhergehende Strahlenbelastung für den Patienten steht einer breiten Anwendung des Verfahrens entgegen. Zudem ist das Verfahren vergleichsweise zeit- und kostenintensiv.

Bei einem weiteren bekannten Verfahren erfolgt die Positionskontrolle elektrokardiographisch (z.B. W. Schummer et al.: "Optimierte Positionierung zentraler Venenkatheter durch eine modifizierte Anwendung der intravasalen Elektrokardiographie", Anästhesist 54 (2005), Seiten 983-990). Dabei wird ein EKG-Signal zwischen einer an der Körperoberfläche des Patienten angebrachten Hautelektrode und einem Seldinger-Draht des Katheters abgeleitet. Das bekannte Verfahren macht es sich zunutze, dass die P-Welle des EKG-Signals sich in Abhängigkeit des Vorschubs der Katheterspitze verändert. Die P-Welle repräsentiert die elektrische Erregung des Vorhofs. Mit Eintreten der Katheterspitze in den Vorhof kommt es zu einer charakteristischen Veränderung der P-Welle. Die Positionskontrolle erfolgt somit über eine Beobachtung des abgeleiteten EKG-Signals. Das bekannte elektrokardiographische Verfahren erfordert zum einen das Anbringen der Hautelektrode an der Körperoberfläche des Patienten. Zudem muss ein EKG-Gerät nebst Monitor zum Beobachten des EKG-Signals bereitgestellt werden.

US 2018/132877 A1 offenbart Navigations- und Gewebeerfassungssysteme und -methoden für die Navigation zu und/oder die Erfassung von ausgewähltem Gewebe unter Verwendung der angeborenen elektrischen Aktivität des ausgewählten Gewebes und/oder anderer Gewebe.

US 2008/097232 A1 offenbart ein Verfahren zur Lokalisierung einer Spitze eines zentralen Venenkatheters (ZVK) mit einem distalen und einem proximalen Elektrodenpaar, die in der oberen Hohlvene, dem rechten Vorhof und/oder dem rechten Ventrikel angeordnet sind. Das Verfahren umfasst die Gewinnung eines distalen und eines proximalen elektrischen Signals von dem distalen und dem proximalen Elektrodenpaar und die Verwendung dieser Signale zur Erzeugung einer distalen bzw. proximalen P-Welle. Für jede der P-Wellen wird ein Ablenkungswert bestimmt. Ein Verhältnis der Ablenkungswerte wird dann verwendet, um die Position der Spitze des ZVK zu bestimmen.

US 2019/038897 A1 offenbart ein Verfahren zum Positionieren eines intravaskulären Katheters, wobei der Katheter eine Vielzahl von Elektroden umfasst und mehrere Elektroden der Vielzahl von Elektroden so konfiguriert sind, dass sie elektrische Signale aussenden.

Aufgabe der Erfindung ist es, ein medizinisches System der eingangs genannten Art bereitzustellen, das Vorteile gegenüber dem Stand der Technik bietet.

Zur Lösung dieser Aufgabe stellt die Erfindung ein medizinisches System mit den Merkmalen des Anspruchs 1 bereit. Vorteilhafte Ausgestaltungen sind in den abhängigen Ansprüchen angegeben. Der Wortlaut sämtlicher Ansprüche wird durch Bezugnahme zum Inhalt der Beschreibung gemacht.

Ein mittels des erfindungsgemäßen medizinischen Systems ausführbares Verfahren zur Kontrolle einer Position und einer Orientierung einer Katheterspitze eines Katheters, insbesondere eines Zentralvenenkatheters, im Körper eines Patienten weist die Schritte auf: a) Erfassen eines ersten elektrischen Potentials mittels einer an der Katheterspitze angeordneten ersten Elektrode und Erzeugen eines ersten Signals, welches einen Zeitverlauf des erfassten ersten elektrischen Potentials repräsentiert; b) Erfassen eines zweiten elektrischen Potentials mittels einer an der Katheterspitze angeordneten sowie proximal von der ersten Elektrode beabstandeten zweiten Elektrode und Erzeugen eines zweiten Signals, welches einen Zeitverlauf des erfassten zweiten elektrischen Potentials repräsentiert; c) Erzeugen eines Differenzsignals in Abhängigkeit des ersten Signals und des zweiten Signals, wobei das Differenzsignal einen Zeitverlauf einer elektrischen Spannung zwischen der ersten Elektrode und der zweiten Elektrode repräsentiert; d) Kontrollieren der Position und der Orientierung der Katheterspitze mittels Vergleichens des Differenzsignals und eines Referenzsignals, welches eine angestrebte Position und Orientierung der Katheterspitze repräsentiert. Die Erfindung geht von der Überlegung aus, dass Katheter, insbesondere Zentralvenenkatheter, oftmals nicht im operativen Umfeld angelegt werden. Mit anderen Worten ist der Patient beim Anlegen des Katheters oftmals nicht per Hautelektroden an ein EKG-Gerät angeschlossen. Das Applizieren von Hautelektroden ist zeitaufwändig. Das Bereitstellen eines EKG-Geräts allein zur Positionskontrolle bedeutet einen apparativen Mehraufwand. Durch die erfindungsgemäße Lösung kann insbesondere auf das Anbringen von Hautelektroden auf der Körperoberfläche des Patienten verzichtet werden. Hierdurch kann zum einen Zeit eingespart werden. Dies ist insbesondere bei medizinischen Notfällen von großem Vorteil. Zum anderen kann der apparative Mehraufwand für das Bereitstellen des EKG-Geräts entfallen. Hierdurch können Kosten eingespart werden. Im Unterschied zu dem bekannten elektrokardiographischen Verfahren sieht die Erfindung insbesondere keine Ableitung von Signalen an der Hautoberfläche des Patienten vor. Stattdessen werden, vorzugsweise ausschließlich, katheterseitige Signale abgeleitet, nämlich das erste Signal und das zweite Signal. Hierfür sind die erste und zweite Elektrode an der Katheterspitze angeordnet. Das erste und zweite Signal repräsentieren das elektrische Potential an der jeweiligen Elektrode, wobei die elektrischen Potentiale aus der elektrischen Aktivität der Herzmuskelzellen resultieren. Die Erfinder haben insbesondere erkannt: Solange sich die erste Elektrode und die zweite Elektrode in einer Vene, beispielsweise der oberen Hohlvene, vor dem rechten Vorhof befinden, haben beide Elektroden einen ähnlichen elektrischen Potentialverlauf. Das heißt die elektrische Spannung zwischen der ersten Elektrode und der zweiten Elektrode ist klein. Sobald die distal an der Katheterspitze angeordnete erste Elektrode in den Vorhof wandert, ändert sich die Potentialdifferenz zwischen der ersten Elektrode und der zweiten Elektrode. Somit ändert sich auch das erzeugte Differenzsignal. Vereinfacht ausgedrückt werden ein charakteristischer Zeitverlauf und/oder eine Amplitudenänderung des Differenzsignals als Messsignal zur Kontrolle der Position verwendet. Weiterhin kann das Differenzsignal, d.h. die Spannung zwischen der ersten Elektrode und der zweiten Elektrode, zur Kontrolle der Orientierung der Katheterspitze verwendet werden. Denn sobald die Katheterspitze mitsamt der ersten Elektrode und der zweiten Elektrode sich vom Herzen wegbewegt, ändert sich eine Polarität des Differenzsignals. Dabei zeigt die Polarität des Differenzsignals an, ob sich die Katheterspitze zum Herzen hin- oder von diesem wegbewegt. Somit kann bereits beim Anlegen des Katheters erkannt werden, ob die Katheterspitze - plakativ ausgedrückt - statt in Richtung Herz in Richtung Kopf abbiegt.

Die Lösung eignet sich in besonders vorteilhafter Weise für Zentralvenenkatheter, PICCs (Peripherally Inserted Central Venous Catheter) sowie sogenannte Midlines. Die erfindungsgemäße Lösung ist jedoch nicht auf solche Katheter eingeschränkt, sondern beispielsweise auch vorteilhaft für Pulmonaliskatheter, Dialysekatheter und/oder arterielle Katheter geeignet.

In einer Ausgestaltung weist das Verfahren den Schritt auf: Ausgeben des Differenzsignals und/oder eines Kontrollsignals, welches eine Abweichung zwischen dem Differenzsignal und dem Referenzsignal repräsentiert. Mit anderen Worten repräsentiert das Kontrollsignal eine Abweichung zwischen der tatsächlichen Position und/oder Orientierung und der angestrebten Position und/oder Orientierung. Das Differenzsignal und/oder das Kontrollsignal wird vorzugsweise für medizinisches Personal optisch und/oder akustisch wahrnehmbar ausgegeben. Dies ist besonders anwenderfreundlich und erlaubt eine einfache und zuverlässige Kontrolle der Position und/oder Orientierung.

Gemäß der Erfindung umfasst das Kontrollieren der Orientierung der Katheterspitze den Schritt: Erfassen und Auswerten eines Vorzeichenwechsels des Differenzsignals, wobei der Vorzeichenwechsel einen Wechsel einer Polarität der ersten Elektrode und der zweiten Elektrode repräsentiert. Die Erfinder haben erkannt, dass die Polarität der ersten Elektrode und/oder der zweiten Elektrode einen Rückschluss auf die Orientierung der Katheterspitze in Relation zum Herzen zulässt. Mit anderen Worten gibt die Polarität der elektrischen Spannung zwischen der ersten Elektrode und der zweiten Elektrode an, ob die Katheterspitze zum Herzen hin- oder von diesem wegorientiert ist. Das Erfassen und Auswerten des hiermit einhergehenden Vorzeichenwechsels des Differenzsignals ist mit einfachen Mitteln möglich. Die Orientierung der Katheterspitze kann durch diese Ausgestaltung der Erfindung mit vergleichsweise geringem apparativem Aufwand kontrolliert werden.

In weiterer Ausgestaltung wird das erste Signal mittels eines ersten Leiterdrahts von der Katheterspitze abgeleitet und/oder das zweite Signal wird mittels eines zweiten Leiterdrahts von der Katheterspitze abgeleitet. Eine solche drahtgebundene Ableitung der Signale ist mit technisch einfachen Mitteln möglich. Der erste Leiterdraht und/oder der zweite Leiterdraht sind ausgehend von der jeweiligen Elektrode in Richtung eines proximalen Endes des Katheters erstreckt. Vorzugsweise sind der erste Leiterdraht und/oder der zweite Leiterdraht im Inneren des Katheters längserstreckt. An ihren der Katheterspitze abgewandten, distalen Enden sind der erste Leiterdraht und/oder der zweite Leiterdraht signalübertragend mit einer zur Signalverarbeitung eingerichteten Auswerteeinrichtung verbunden. Bei dieser Ausgestaltung der Erfindung können die erste Elektrode und/oder die zweite Elektrode durch ein distales Ende des jeweiligen Leiterdrahts gebildet sein.

In weiterer Ausgestaltung wird das erste Signal über ein flüssigkeitsbefülltes erstes Katheterlumen von der Katheterspitze abgeleitet und/oder das zweite Signal wird über ein flüssigkeitsbefülltes zweites Katheterlumen von der Katheterspitze abgeleitet. Durch diese Ausgestaltung der Erfindung kann insbesondere auf eine drahtgebundene Ableitung des ersten Signals und/oder des zweiten Signals verzichtet werden. Das erste Katheterlumen und/oder das zweite Katheterlumen ist in der eigentlichen Anwendung des Katheters zum Zuführen einer Medikamentenflüssigkeit und/oder zum Entnehmen von Blut aus dem Venensystem des Patienten vorgesehen. Hierdurch kommt dem ersten und/oder zweiten Katheterlumen bei dieser Ausgestaltung der Erfindung eine besonders vorteilhafte Mehrfachfunktion zu. Damit wird der Aufbau des Katheters vereinfacht. Dies erlaubt letztlich eine einfache und kostengünstige Herstellbarkeit. Bei dieser Ausgestaltung der Erfindung können die erste Elektrode und/oder die zweite Elektrode durch eine distale Stirnendöffnung des jeweiligen Katheterlumens gebildet sein. Hierdurch kann ein nochmals vereinfachter Aufbau des Katheters erreicht werden.

In weiterer Ausgestaltung weist das Verfahren die Schritte auf: Erfassen eines dritten elektrischen Potentials mittels einer an der Katheterspitze angeordneten sowie proximal von der zweiten Elektrode beabstandeten dritten Elektrode und Erzeugen eines dritten Signals, welches einen Zeitverlauf des erfassten dritten elektrischen Potentials repräsentiert; Erzeugen eines weiteren Differenzsignals in Abhängigkeit des zweiten Signals und des dritten Signals, wobei das weitere Differenzsignal einen Zeitverlauf einer elektrischen Spannung zwischen der zweiten Elektrode und der dritten Elektrode repräsentiert; wobei das Kontrollieren der Position und/oder der Orientierung der Katheterspitze ein Vergleichen des Differenzsignals und des weiteren Differenzsignals umfasst. Vereinfacht ausgedrückt wird bei dieser Ausgestaltung der Erfindung das elektrische Potential an einer zusätzlichen Stelle im Bereich der Katheterspitze erfasst. Zu diesem Zweck ist die dritte Elektrode vorgesehen und proximal von der zweiten Elektrode beabstandet. Die Erfinder haben erkannt, dass hierdurch eine höhere Sicherheit erreicht werden kann. Das weitere Differenzsignal und das Differenzsignal werden zur Kontrolle der Position und/oder Orientierung miteinander verglichen. Dieser Vergleich kann mittels entsprechender Algorithmen erfolgen, beispielsweise indem das weitere Differenzsignal von dem Differenzsignal subtrahiert wird oder umgekehrt. Das dritte Signal kann auf eine dem ersten und/oder dem zweiten Signal entsprechende Weise von der Katheterspitze abgeleitet werden. Beispielsweise kann hierfür ein dritter Leiterdraht oder ein flüssigkeitsbefülltes drittes Katheterlumen vorgesehen sein. Die dritte Elektrode kann dementsprechend durch ein distales Stirnende des dritten Leiterdrahts oder durch eine distale Stirnendöffnung des dritten Katheterlumens gebildet sein. Es versteht sich, dass bei weiteren Ausführungsformen der Erfindung mehr als lediglich das erste, zweite und dritte elektrische Potential an der Katheterspitze erfasst werden können. Dementsprechend können mehr als die erste, zweite und dritte Elektrode an der Katheterspitze angeordnet sein. Denkbar ist beispielsweise die Anordnung von vier, fünf, sechs, mehr als sechs oder mehr als zehn Elektroden.

Das erfindungsgemäße medizinisches System weist auf: einen Katheter, insbesondere Zentralvenenkatheter, mit einer Katheterspitze, an welcher wenigstens eine erste Elektrode und eine zweite Elektrode angeordnet sind, wobei die zweite Elektrode in proximaler Richtung von der ersten Elektrode beabstandet ist, wobei die erste Elektrode zum Erfassen eines ersten elektrischen Potentials und zum Erzeugen eines ersten Signals eingerichtet ist, welches einen Zeitverlauf des ersten elektrischen Potentials repräsentiert, und wobei die zweite Elektrode zum Erfassen eines zweiten elektrischen Potentials und zum Erzeugen eines zweiten Signals eingerichtet ist, welches einen Zeitverlauf des zweiten elektrischen Potentials repräsentiert; eine Auswerteeinrichtung, die mit der ersten Elektrode und der zweiten Elektrode verbunden ist, wobei die Auswerteeinrichtung eingerichtet ist zum Erzeugen eines Differenzsignals in Abhängigkeit des ersten Signals und des zweiten Signals, wobei das Differenzsignal einen Zeitverlauf einer elektrischen Spannung zwischen der ersten Elektrode und der zweiten Elektrode repräsentiert. Das erfindungsgemäße medizinische System ermöglicht eine apparativ unaufwändige, zeitsparende und zuverlässige Kontrolle der Position und/oder Orientierung der Katheterspitze. Im Übrigen wird zur Vermeidung von Wiederholungen auf die Offenbarung zu dem mittels des erfindungsgemäßen medizinischen Systems ausführbaren Verfahren verwiesen. Das dort im Hinblick auf mit der erfindungsgemäßen Lösung einhergehenden Vorteile Gesagte gilt sinngemäß für das erfindungsgemäße medizinische System. Der Katheter ist insbesondere als Zentralvenenkatheter, PICC (Peripherally Inserted Central Venous Catheter), Midline, Pulmonaliskatheter, Dialysekatheter oder arterieller Katheter gestaltet. Die erste Elektrode und die zweite Elektrode sind im Bereich der Katheterspitze angeordnet und vorzugsweise jeweils auf einer Außenmantelfläche der Katheterspitze angeordnet und/oder ausgebildet. Die Auswerteeinrichtung ist zum Auswerten der mittels der ersten und zweiten Elektrode erfassten und/oder erzeugten ersten und zweiten Signale eingerichtet. Insbesondere ist die Auswerteeinrichtung zum Erzeugen des Differenzsignals in Abhängigkeit des ersten und des zweiten Signals eingerichtet. Die Auswerteeinrichtung kann drahtgebunden und/oder drahtlos mit der ersten Elektrode und der zweiten Elektrode verbunden sein. Zur drahtgebundenen Verbindung weist der Katheter wenigstens einen ersten Leiterdraht, der der ersten Elektrode zugeordnet ist, und/oder einen zweiten Leiterdraht, der der zweiten Elektrode zugeordnet ist. Alternativ oder zusätzlich ist eine drahtlose Verbindung über ein erstes Katheterlumen, das der ersten Elektrode zugeordnet ist, und/oder ein zweites Katheterlumen vorgesehen, das der zweiten Elektrode zugeordnet ist. In der eigentlichen Anwendung des Katheters ist das erste Katheterlumen und/oder das zweite Katheterlumen mit (elektrisch leitfähiger) Flüssigkeit befüllt. Dies ermöglicht eine drahtlose Signalableitung.

Weiter gemäß der Erfindung ist die Auswerteeinrichtung eingerichtet zum Vergleichen des Differenzsignals mit einem Referenzsignal, welches eine angestrebte Position und Orientierung der Katheterspitze repräsentiert. Mit anderen Worten ist die Auswerteeinrichtung eingerichtet zum Auswerten eines Kurvenverlaufs des Differenzsignals. Eine signifikante Änderung des Kurvenverlaufs lässt einen Rückschluss auf die Position und Orientierung der Katheterspitze zu.

In weiterer Ausgestaltung der Erfindung ist eine mit der Auswerteeinrichtung verbundene Ausgabeeinrichtung vorgesehen, die zum Ausgeben des Differenzsignals und/oder eines Kontrollsignals eingerichtet ist, welche eine Abweichung zwischen dem Differenzsignal und dem Referenzsignal repräsentiert. Die Ausgabeeinrichtung kann zum optischen und/oder akustischen Ausgeben des betreffenden Signals eingerichtet sein. Die Ausgabeeinrichtung ist vorzugsweise in die Auswerteeinrichtung integriert oder umgekehrt. Die Ausgabeeinrichtung kann ein Display, einen Bildschirm, ein Projektionsgerät oder dergleichen aufweisen.

In weiterer Ausgestaltung der Erfindung ist wenigstens eine weitere, dritte Elektrode an der Katheterspitze angeordnet und in proximaler Richtung von der zweiten Elektrode beabstandet, wobei die dritte Elektrode zum Erfassen eines dritten elektrischen Potentials und zum Erzeugen eines dritten Signals eingerichtet ist, welches einen Zeitverlauf des erfassten dritten elektrischen Potentials repräsentiert, wobei die Auswerteeinrichtung mit der dritten Elektrode verbunden und eingerichtet ist zum Erzeugen eines weiteren Differenzsignals in Abhängigkeit des zweiten Signals und des dritten Signals, wobei das weitere Differenzsignal einen Zeitverlauf einer elektrischen Spannung zwischen der zweiten Elektrode und der dritten Elektrode repräsentiert, und wobei die Auswerteeinrichtung eingerichtet ist zum Vergleichen des weiteren Differenzsignals und des Differenzsignals.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematisch vereinfachter Diagrammdarstellung einzelne Schritte einer Ausführungsform eines Verfahrens,
- Fig. 2: eine schematisch stark vereinfachte Darstellung einer Ausführungsform eines erfindungsgemäßen medizinischen Systems, das zum Ausführen einer Ausführungsform des Verfahrens eingerichtet ist und einen Katheter, eine Auswerteeinrichtung und eine Ausgabeeinrichtung aufweist,
- Fig. 3: eine schematische Detaildarstellung der Katheterspitze des Katheters nach Fig. 2, wobei eine erste und zweite Elektrode an der Katheterspitze angeordnet sind,
- Fig. 4 bis 6: unterschiedliche Anwendungssituationen bei einem Anlegen des Katheters nach Fig. 2 zur Verdeutlichung des Verfahrens nach Fig. 1,
- Fig. 7: eine weitere Anwendungssituation zur Verdeutlichung des Verfahrens, wobei zwei unterschiedliche Positionen und/oder Orientierungen der Katheterspitze überlagert dargestellt sind,
- Fig. 8: schematische Darstellungen unterschiedlicher Signalverläufe, die der anhand Fig. 7 gezeigten Anwendungssituation zugeordnet sind und die dort gezeigten unterschiedlichen Orientierungen der Katheterspitze repräsentieren,
- Fig. 9: eine weitere Ausführungsform eines erfindungsgemäßen medizinischen Systems, wobei eine zusätzliche, dritte Elektrode an der Katheterspitze angeordnet ist, und
- Fig. 10 bis 12: unterschiedliche Anwendungssituationen beim Anlegen des Katheters des medizinischen Systems nach Fig. 9 in einer den Fig. 4 bis 6 entsprechenden Darstellungsweise.

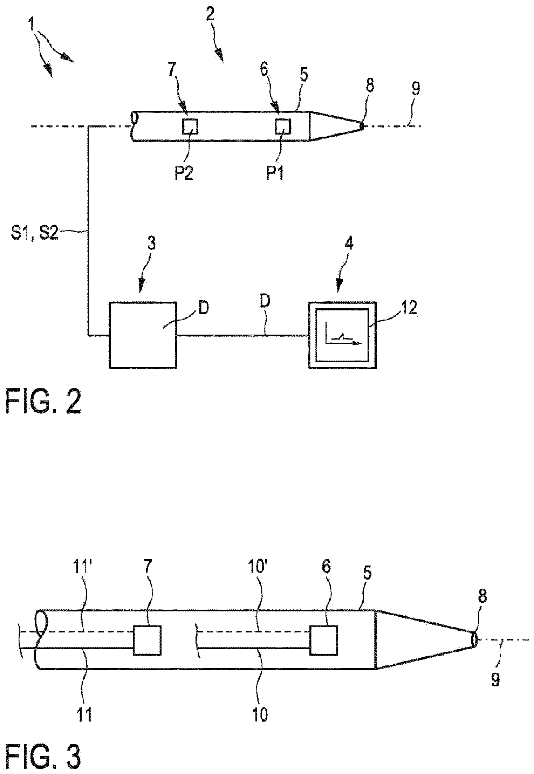

Gemäß Fig. 2 ist ein medizinisches System 1 mit einem Katheter 2, einer Auswerteeinrichtung 3 und einer Ausgabeeinrichtung 4 vorgesehen. Das medizinische System 1 ist anhand Fig. 2 schematisch stark vereinfacht dargestellt.

Der Katheter 2 ist lediglich im Bereich seiner Katheterspitze 5 gezeigt. Die Katheterspitze 5 ist an einem distalen Ende des Katheters 2 angeordnet. Vorliegend handelt es sich bei dem Katheter 2 um einen Zentralvenenkatheter.

Bei zeichnerisch nicht dargestellten Ausführungsformen kann der Katheter stattdessen ein PICC-Katheter, eine Midline, ein Pulmonaliskatheter, ein Dialysekatheter oder ein arterieller Katheter sein.

Der Katheter 2 weist im Bereich seiner Katheterspitze 5 eine erste Elektrode 6 und eine zweite Elektrode 7 auf. Die erste Elektrode 6 ist vergleichsweise näher an einem distalen Stirnende 8 der Katheterspitze 5 angeordnet als die zweite Elektrode 7. Mit anderen Worten ist die zweite Elektrode 7 in proximaler Richtung von der ersten Elektrode 6 beabstandet an der Katheterspitze 5 angeordnet. Dabei ist die zweite Elektrode 7 entlang einer zwischen dem distalen Stirnende 8 und einem nicht dargestellten proximalen Stirnende erstreckten Längsachse 9 von der ersten Elektrode 6 beabstandet. Die erste und zweite Elektrode 6, 7 sind jeweils zum Erfassen eines elektrischen Potentials eingerichtet. Das an der ersten Elektrode 6 erfassbare oder erfasste elektrische Potential kann auch als erstes elektrisches Potential P1 bezeichnet werden. Das der zweiten Elektrode 7 zugeordnete elektrische Potential kann dementsprechend als zweites elektrisches Potential P2 bezeichnet werden.

Bei der gezeigten Ausführungsform sind die erste und zweite Elektrode 6, 7 jeweils zum Erzeugen eines Signals eingerichtet, welches einen Zeitverlauf des jeweils erfassten elektrischen Potentials repräsentiert. Insoweit ist die erste Elektrode 6 zum Erzeugen eines ersten Signals S1 eingerichtet. Die zweite Elektrode 7 ist zum Erzeugen eines zweiten Signals S2 eingerichtet. Das erste Signal S1 repräsentiert den Zeitverlauf des mittels der ersten Elektrode 6 erfassten ersten elektrischen Potentials P1. Dementsprechend repräsentiert das zweite Signal S2 den Zeitverlauf des mittels der zweiten Elektrode 7 erfassten zweiten elektrischen Potentials P2.

Alternativ oder zusätzlich kann die Auswerteeinrichtung 3 zum Erzeugen des ersten und zweiten Signals S1, S2 in Abhängigkeit der mittels der ersten und zweiten Elektrode 6, 7 erfassten ersten und zweiten elektrischen Potentiale P1, P2 eingerichtet sein.

Die erste und zweite Elektrode 6, 7 sind jeweils mit der Auswerteeinrichtung 3 verbunden. Diese Verbindung dient einer Übertragung der mittels der Elektroden 6, 7 erfassten elektrischen Potentiale P1, P2 und/oder der Signale S1, S2, sofern letztere mittels der Elektrode 6, 7 erzeugt werden. Diese Übertragung kann gemäß einem in der Medizin üblichen Sprachgebrauch auch als Ableitung bezeichnet werden.

Wie anhand Fig. 3 gezeigt ist, kann die Ableitung drahtgebunden oder drahtlos erfolgen. Dabei verdeutlicht Fig. 3 beide Varianten, wobei vorzugsweise entweder eine drahtgebundene oder eine drahtlose Ableitung vorgesehen ist.

Zur drahtgebundenen Ableitung sind ein erster Leiterdraht 10 und ein zweiter Leiterdraht 11 vorgesehen. Der erste Leiterdraht 10 ist der ersten Elektrode 6 zugeordnet. Der zweite Leiterdraht 11 ist der zweiten Elektrode 7 zugeordnet. Die Leiterdrähte 10, 11 stellen eine elektrisch leitfähige Verbindung zwischen den Elektroden 6, 7 einerseits und der Auswerteeinrichtung 3 andererseits her. Vorzugsweise sind die Leiterdrähte 10, 11 im Inneren des Katheters 2 längserstreckt. Dabei ist die anhand Fig. 3 gezeigte Anordnung als rein exemplarisch und schematisch stark vereinfacht anzusehen.

Die Elektroden 6, 7 können als separate Bauteile und/oder Abschnitte gefertigt und hiernach elektrisch leitend mit dem jeweiligen Leiterdraht 10, 11 verbunden sein. Alternativ können die Elektroden 6, 7 durch ein distales Stirnende des jeweiligen Leiterdrahts 10, 11 gebildet sein.

Zur drahtlosen Ableitung kann der Katheter 2 ein der ersten Elektrode 6 zugeordnetes erstes Katheterlumen 10' aufweisen. Der zweiten Elektrode 7 kann dementsprechend ein zweites Katheterlumen 11' zugeordnet sein. In der Anwendung des Katheters 2 sind die Katheterlumen 10', 11' auf eine dem Fachmann grundsätzlich bekannte Weise zur Flüssigkeitsleitung vorgesehen. Beispielsweise kann mittels der Katheterlumen 10', 11' eine medizinische Flüssigkeit verabreicht oder Blut entnommen werden. Vorliegend dienen die Katheterlumen 10', 11' zusätzlich der besagten Ableitung, wobei diese über die während der Anwendung des Katheters 2 in den Katheterlumen 10', 11' befindliche elektrisch leitfähige Flüssigkeit erfolgt. Die Katheterlumen 10', 11' sind anhand Fig. 3 schematisch stark vereinfacht und strichliert dargestellt. Die erste Elektrode 6 kann durch eine distale Stirnendöffnung des ersten Katheterlumens 10' gebildet sein. Die zweite Elektrode 7 kann durch eine distale Stirnendöffnung des zweiten Katheterlumens 11' gebildet sein.

Die Auswerteeinrichtung 3 ist zur Signalauswertung der mittels der Elektroden 6, 7 erfassten bzw. erzeugten Signale S1, S2 eingerichtet. Die Signalauswertung umfasst bei der gezeigten Ausführungsform das Erzeugen eines Differenzsignals D. Das Differenzsignal D wird in Abhängigkeit des ersten Signals S1 und des zweiten Signals S2 erzeugt und repräsentiert eine zwischen der ersten Elektrode 6 und der zweiten Elektrode 7 vorherrschende elektrische Spannung (Potentialdifferenz zwischen dem ersten elektrischen Potential P1 und dem zweiten elektrischen Potential P2).

Bei der gezeigten Ausführungsform ist die Auswerteeinrichtung 3 mit der Ausgabeeinrichtung 4 verbunden. Die Ausgabeeinrichtung 4 ist vorliegend zur optischen Ausgabe des Differenzsignals D eingerichtet. Vorliegend weist die Ausgabeeinrichtung 4 hierfür einen Monitor 12 auf. Anhand Fig. 2 ist mittels des Monitors 12 ein exemplarischer Zeitverlauf des Differenzsignals D schematisch dargestellt.

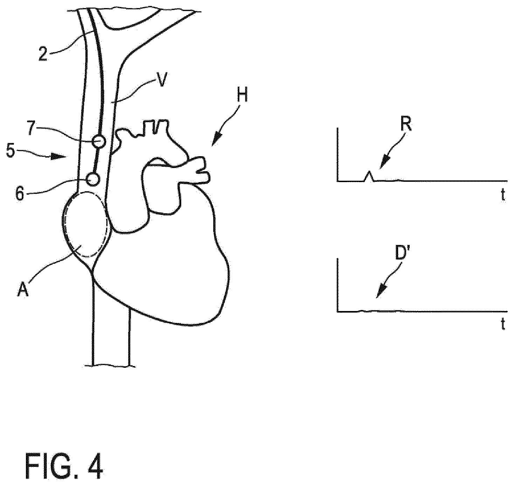

Ein Anlegen des Katheters 2 erfolgt auf eine dem Fachmann grundsätzlich bekannte Weise. Dabei wird der Katheter 2 üblicherweise über eine Vene der oberen Körperhälfte eines Patienten in dessen Venensystem eingeführt. Die Katheterspitze 5 wird bis in den Bereich des rechten Vorhofs des Herzens H vorgeschoben (Fig. 4 bis 6). Der rechte Vorhof wird nachfolgend als Atrium A bezeichnet. Es ist bekannt, dass eine nicht ausreichend genaue Positionierung der Katheterspitze 5 in Relation zum Atrium A problematisch ist. Um Probleme zu vermeiden, ist daher eine Kontrolle der Position der Katheterspitze 5 erforderlich. Das medizinische System 1 erlaubt eine solche Kontrolle. Zur näheren Erläuterung wird nachfolgend insbesondere auf die Fig. 4 bis 6 abgestellt.

Die Fig. 4 bis 6 zeigen exemplarisch unterschiedliche Situationen einem Anlegen des Katheters 2.

Fig. 4 zeigt eine erste Situation, in welcher die Katheterspitze 5 auf grundsätzlich bekannte Weise über die obere Hohlvene V bis kurz vor das Atrium A vorgeschoben ist. Die anhand Fig. 4 gezeigte Position der Katheterspitze 5 kann auch als erste Position bezeichnet werden. In dieser ist sowohl die erste Elektrode 6 als auch die zweite Elektrode 7 außerhalb des Atriums A angeordnet.

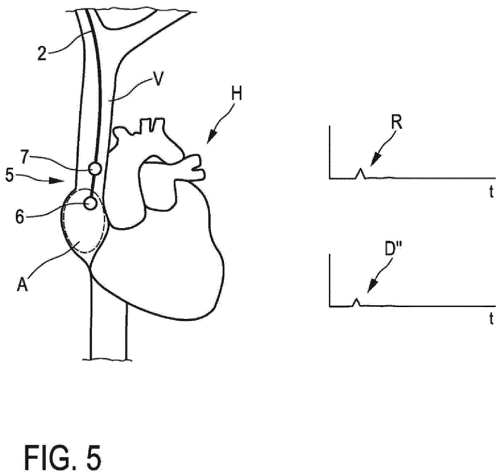

Fig. 5 zeigt eine zweite Situation, in welcher die Katheterspitze 5 ausgehend von der ersten Position weitergehend in Richtung des Atriums A vorgeschoben ist. Die anhand Fig. 5 gezeigte Position der Katheterspitze 5 kann auch als zweite Position bezeichnet werden. In dieser befindet sich die erste Elektrode 6 innerhalb des Atriums A. Dementgegen ist die zweite Elektrode 7 außerhalb des Atriums A in der oberen Hohlvene V angeordnet.

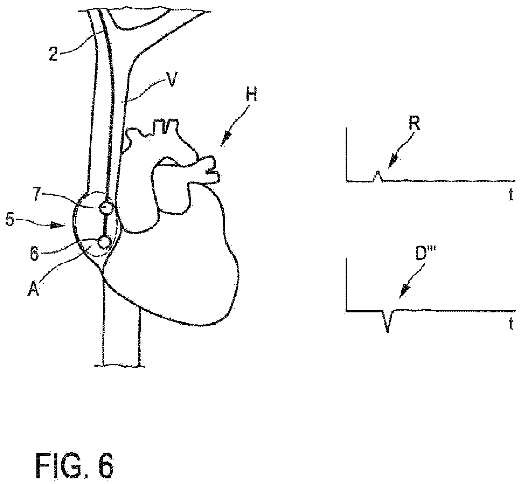

Anhand Fig. 6 ist exemplarisch eine dritte Situation gezeigt. In dieser ist die Katheterspitze 5 ausgehend von der zweiten Position weiter vorgeschoben. Die anhand Fig. 6 gezeigte Position der Katheterspitze 5 kann auch als dritte Position bezeichnet werden. In der dritten Position befinden sich die erste Elektrode 6 und die zweite Elektrode 7 innerhalb des Atriums A.

Bei dem Anlegen des Katheters und der damit einhergehenden Vorschubbewegung der Katheterspitze 5 werden die elektrischen Potentiale P1, P2 und damit die durch das Differenzsignal D repräsentierte elektrische Spannung zwischen den Elektroden 6, 7, vorzugsweise kontinuierlich, erfasst.

Die Erfinder haben erkannt, dass sich die elektrische Spannung - und damit das Differenzsignal D - in Abhängigkeit der Position der Katheterspitze 5 in Relation zu dem Atrium A verändert. Die Kontrolle der Position der Katheterspitze 5 kann - vereinfacht ausgedrückt - auf Grundlage der erfassten elektrischen Spannung und damit des Differenzsignals D erfolgen.

Solange sich die erste Elektrode 6 und die zweite Elektrode 7 in der Hohlvene V vor dem Atrium A befinden, liegt an beiden Elektroden 6, 7 ein ähnliches elektrisches Potential an. Mit anderen Worten ist die elektrische Spannung in der ersten Position (Fig. 4) vergleichsweise klein. Dies wird durch den in Fig. 4 gezeigten exemplarischen Zeitverlauf eines sich hierbei in der ersten Situation ergebenden Differenzsignals D' verdeutlicht. Der Zeitverlauf des Differenzsignals D' zeigt keinen nennenswerten Ausschlag.

Sobald die erste Elektrode 6 in das Atrium A eintritt (Fig. 5), kommt es zu einer Änderung der mittels der Elektroden 6, 7 erfassten elektrischen Potentiale und damit einhergehend der zwischen den Elektroden 6, 7 anliegenden Spannung. Dementsprechend ändert sich der Zeitverlauf des Differenzsignals. Dies ist in Fig. 5 durch ein Differenzsignal D" verdeutlicht. Das Differenzsignal D" weist gegenüber dem Differenzsignal D' in der ersten Position (Fig. 4) einen geänderten Signalverlauf über der Zeit auf.

In der dritten Position (Fig. 6) kommt es zu einer nochmaligen Änderung der Spannung zwischen der ersten Elektrode 6 und der zweiten Elektrode 7. Dementsprechend ändert sich der Zeitverlauf des Differenzsignals, was anhand Fig. 4 exemplarisch durch das Differenzsignal D‴ gezeigt ist.

Die durch die exemplarischen Kurvenverläufe D', D" und D‴ symbolisierte charakteristische Änderung des Zeitverlaufs des Differenzsignals D lässt einen Rückschluss auf die Position der Katheterspitze 5 in Relation zu dem Atrium A zu. Hierdurch kann die Position der Katheterspitze 5 kontrolliert werden.

Die Kontrolle umfasst bei der gezeigten Ausführungsform ein Vergleichen des Differenzsignals D mit einem Referenzsignal R. Das Referenzsignal R repräsentiert eine angestrebte Position und/oder Orientierung der Katheterspitze 5.

Das Vergleichen des Differenzsignals D und des Referenzsignals R kann beispielsweise durch medizinisches Personal visuell mittels des Bildschirms 12 erfolgen. Alternativ oder zusätzlich kann das Vergleichen mittels der Auswerteeinrichtung 3 erfolgen.

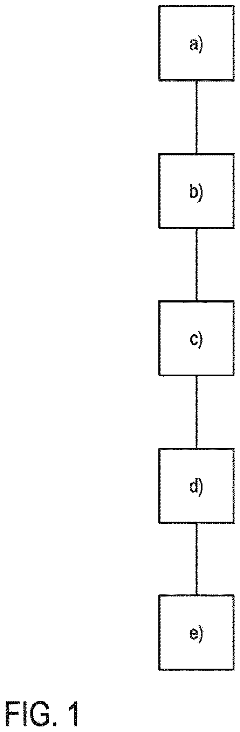

Das zugrunde liegende Verfahren ist anhand Fig. 1 schematisch stark vereinfacht dargestellt und umfasst die Schritte a) bis e). Hierzu kurz im Einzelnen:
Gemäß dem Schritt a) wird das erste Signal S1 auf Grundlage des mittels der ersten Elektrode 6 erfassten ersten elektrischen Potentials P1 erzeugt.

Gemäß dem Schritt b) wird das zweite Signal S2 dementsprechend erzeugt. Dies auf Grundlage des mittels der zweiten Elektrode 7 erfassten zweiten elektrischen Potentials P2.

Gemäß dem Schritt c) wird das Differenzsignal D erzeugt. Dies erfolgt vorliegend mittels der Auswerteeinrichtung 3.

Gemäß dem Schritt d) wird die Position der Katheterspitze 5 kontrolliert. Dies vorliegend mittels des besagten Vergleichs zwischen dem (Zeitverlauf) des Differenzsignals D und des Referenzsignals R. Das Vergleichen kann mittels einer visuellen Kontrolle am Bildschirm 12 erfolgen. Alternativ oder zusätzlich werden das Differenzsignal D und das Referenzsignal R mittels der Auswerteeinrichtung 3, vorzugsweise mittels hierfür geeigneter Algorithmen, miteinander verglichen. Im einfachsten Fall umfasst der Vergleich eine einfache Subtraktion.

Sofern ein visueller Vergleich vorgesehen ist, wird das Differenzsignal D gemäß dem Schritt e) ausgegeben. Alternativ oder zusätzlich kann ein Kontrollsignal, das die Abweichung zwischen dem Differenzsignal D und dem Referenzsignal R repräsentiert, ausgegeben werden.

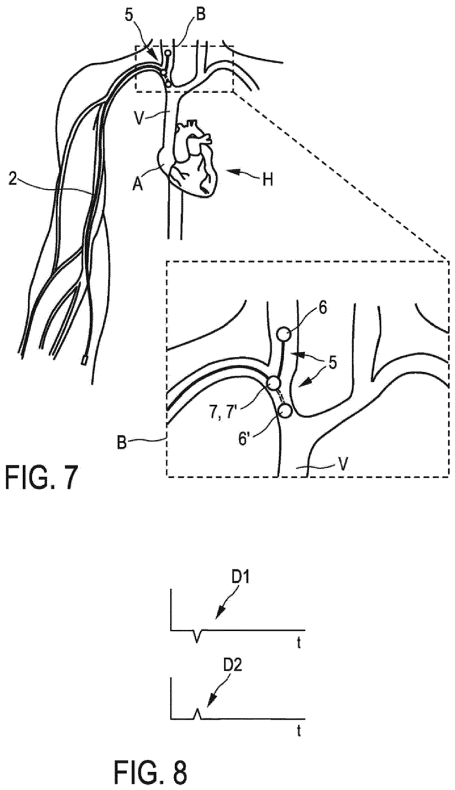

Das medizinische System 1 lässt neben einer Kontrolle der Position alternativ oder zusätzlich auch eine Kontrolle der Orientierung der Katheterspitze 5 zu. Dies ist anhand der Fig. 7 und 8 verdeutlicht. Fig. 7 zeigt zwei zeichnerisch überlagerte Situationen während des Anlegens des Katheters 2. Letzterer ist bei der anhand Fig. 7 gezeigten Ausführungsform als PICC-Katheter gestaltet und dementsprechend ausgehend von der Armbeuge in das Venensystem zugeführt. In beiden dargestellten Situationen ist die Katheterspitze 5 in einem Bereich B oberhalb der oberen Hohlvene V angeordnet.

In der ersten dargestellten Situation ist die Katheterspitze 5 unbeabsichtigterweise nicht in Richtung des Atriums A, sondern stattdessen in Richtung Kopf vorgeschoben. Dieser Situation sind die Bezugszeichen 6 und 7 der ersten und zweiten Elektrode zugeordnet. In der zweiten Situation ist die Katheterspitze 5 stattdessen anforderungsgemäß in Richtung des Atriums A vorgeschoben. Dieser zweiten Situation sind in dem vergrößert dargestellten Bereich B die apostrophierten Bezugszeichen 6' und 7' zugeordnet.

Die Erfinder haben erkannt, dass die Polarität des Differenzsignals sich in Abhängigkeit der Orientierung der Katheterspitze 5 in Relation zu dem Herzen H verändert. Dies ist anhand Fig. 8 verdeutlicht. Dort sind zwei exemplarische Signalverläufe D1, D2 des Differenzsignals D dargestellt. Der Signalverlauf D1 ist der ersten Situation und damit der in Richtung des Kopfs ausgerichteten Orientierung der Katheterspitze 5 zugeordnet. Der zweite Signalverlauf D2 ist der Orientierung der Katheterspitze 5 in Richtung des Herzens H zugeordnet (zweite Situation). Je nach Orientierung der Katheterspitze 5 ist die Polarität des Differenzsignals unterschiedlich. Mit anderen Worten ändert sich das Vorzeichen des Differenzsignals D. Dies ist anhand der Signalverläufe D1, D2 symbolisiert. Hierdurch kann mittels einer entsprechenden Signalauswertung auf einfache Weise die Orientierung der Katheterspitze 5 kontrolliert werden.

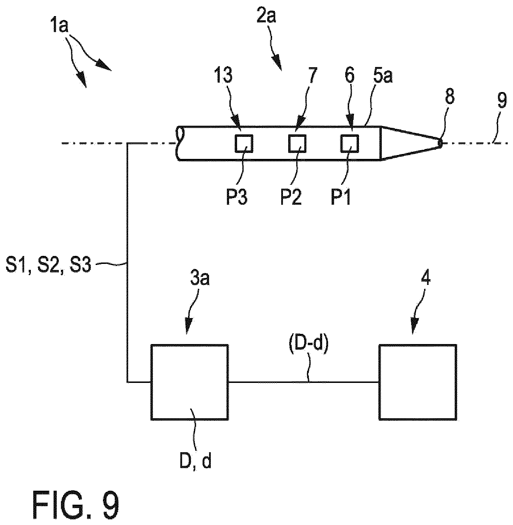

Fig. 9 zeigt ein medizinisches System 1a, das weitestgehend identisch zu dem medizinischen System 1 nach Fig. 2 gestaltet ist. Auch die Funktionsweise ist weitestgehend identisch. Nachfolgend wird lediglich auf wesentliche Unterschiede des medizinischen Systems 1a gegenüber dem medizinischen System 1 nach Fig. 2 eingegangen.

Wesentlicher Unterschied ist die Anzahl der Elektroden an der Katheterspitze 5a. Vorliegend ist an dem Katheter 2a zusätzlich zu der ersten und zweiten Elektrode 5, 7 eine dritte Elektrode 13 vorgesehen. Die dritte Elektrode 13 ist in proximaler Richtung entlang der Längsachse 9 von der zweiten Elektrode 7 beabstandet. Die dritte Elektrode 13 ist zum Erfassen eines dritten elektrischen Potentials P3 und zum Erzeugen eines dritten Signals S3 eingerichtet. Vereinfacht ausgedrückt ist die dritte Elektrode 13 abgesehen von ihrer Anordnung identisch mit der ersten und zweiten Elektrode 6, 7. Zur Ableitung des Signals S3 und der hierfür vorgesehenen Verbindung zwischen der dritten Elektrode 13 und der Auswerteeinrichtung 3a gilt sinngemäß das bereits zur Ableitung der Signale S1, S2 Gesagte (Fig. 3).

Weiter im Unterschied ist die Auswerteeinrichtung 3a zusätzlich zur Verarbeitung des dritten Signals S3 eingerichtet. In Abhängigkeit des dritten Signals S3 und des zweiten Signals S2 wird ein weiteres Differenzsignal d erzeugt. Das weitere Differenzsignal d repräsentiert einen Zeitverlauf einer elektrischen Spannung zwischen der zweiten Elektrode 7 und der dritten Elektrode 13.

Bei der Ausführungsform nach Fig. 9 umfasst das Kontrollieren der Position und/oder der Orientierung der Katheterspitze 5 ein Vergleichen des Differenzsignals D und des weiteren Differenzsignals d. Im einfachsten Fall wird das weitere Differenzsignal d von dem Differenzsignal D subtrahiert. Ein aus dem Vergleich resultierendes Vergleichssignal D-d kann beispielsweise mittels der Ausgabeeinrichtung 4 ausgegeben und der Kontrolle zugrunde gelegt werden.

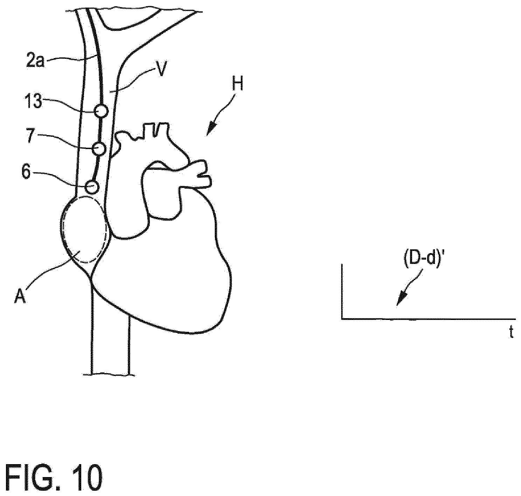

Anhand der Fig. 10 bis 12 sind in Entsprechung zu den Fig. 4 bis 6 unterschiedliche Situationen beim Anlegen des Katheters 2a gezeigt. Die dort dargestellten Signalverläufe symbolisieren unterschiedliche charakteristische Signalverläufe (D-d)', (D-d)", (D-d)‴ des vorbeschriebenen Vergleichssignals.

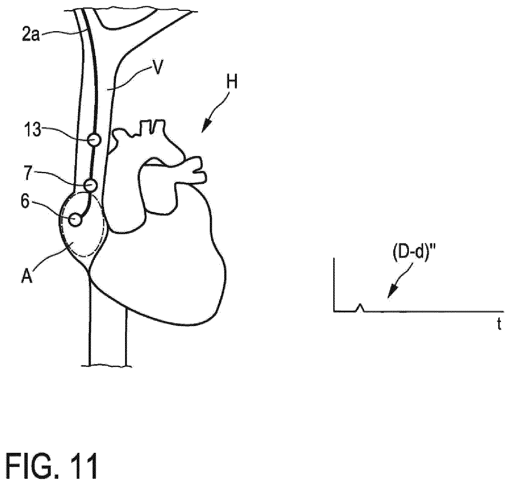

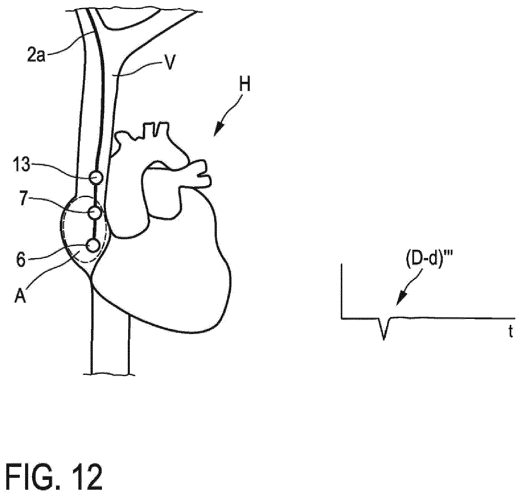

In der ersten Position (Fig. 10) der Katheterspitze 5a (Fig. 9) sind die erste, zweite und dritte Elektrode 6, 7, 13 außerhalb des Atriums A in der oberen Hohlvene V angeordnet. In der zweiten Position (Fig. 11) ist die Katheterspitze 5a weitergehend in Richtung des Atriums A vorgeschoben. Dabei befindet sich die erste Elektrode 6 innerhalb und die zweite und dritte Elektrode 7, 13 befinden sich außerhalb des Atriums A. In der dritten Position (Fig. 12) ist die Katheterspitze 5a weiter vorgeschoben, wobei lediglich die dritte Elektrode 13 noch außerhalb des Atriums A angeordnet ist. Die sich hierbei ergebenden unterschiedlichen Spannungen zwischen den Elektroden 6, 7, 13 resultieren in den exemplarisch dargestellten, unterschiedlichen Signalverläufen. Diese können in Entsprechung zu dem anhand der Fig. 4 bis 6 erläuterten Verfahren der Kontrolle der Position der Katheterspitze zugrunde gelegt werden. Dabei haben die Erfinder erkannt, dass durch die zusätzliche, dritte Elektrode 13 eine höhere Sicherheit bei der Signalerfassung erreicht werden kann. Hierdurch kann letztlich eine zuverlässigere und/oder genauere Kontrolle der Position ermöglicht werden. Entsprechendes gilt im Hinblick auf die Kontrolle der Orientierung.

## Patentansprüche

1. Medizinisches System (1, 1a) zur Kontrolle einer Position und einer Orientierung einer Katheterspitze (5, 5a) im Körper eines Patienten, aufweisend
einen Katheter (2, 2a), insbesondere einen Zentralvenenkatheter, mit der Katheterspitze (5, 5a), an welcher wenigstens eine erste Elektrode (6) und eine zweite Elektrode (7) angeordnet sind, wobei die zweite Elektrode (7) in proximaler Richtung von der ersten Elektrode (6) beabstandet ist, wobei die erste Elektrode (6) zum Erfassen eines ersten elektrischen Potentials (P1) und zum Erzeugen eines ersten Signals (S1) eingerichtet ist, welches einen Zeitverlauf des ersten elektrischen Potentials (P1) repräsentiert, und wobei die zweite Elektrode (7) zum Erfassen eines zweiten elektrischen Potentials (P2) und zum Erzeugen eines zweiten Signals (S2) eingerichtet ist, welches einen Zeitverlauf des zweiten elektrischen Potentials (P2) repräsentiert,
eine Auswerteeinrichtung (3, 3a), die mit der ersten Elektrode (6) und der zweiten Elektrode (7) verbunden ist, wobei die Auswerteeinrichtung (3, 3a) eingerichtet ist zum Erzeugen eines Differenzsignals (D) in Abhängigkeit des ersten Signals (S1) und des zweiten Signals (S2), wobei das Differenzsignal (D) einen Zeitverlauf einer elektrischen Spannung zwischen der ersten Elektrode (6) und der zweiten Elektrode (7) repräsentiert, wobei die Auswerteeinrichtung (3, 3a) eingerichtet ist zum Vergleichen des Differenzsignals (D) mit einem Referenzsignal (R), welches eine angestrebte Position und Orientierung der Katheterspitze (5, 5a) repräsentiert, und wobei die Auswerteeinrichtung (3, 3a) eingerichtet ist zum Erfassen und Auswerten eines Vorzeichenwechsels des Differenzsignals (D), wobei der Vorzeichenwechsel einen Wechsel einer Polarität der ersten Elektrode (6) und der zweiten Elektrode (7) repräsentiert.

2. Medizinisches System (1, 1a) nach Anspruch 1, **gekennzeichnet durch** eine mit der Auswerteeinrichtung (3, 3a) verbundene Ausgabeeinrichtung (4), die zum Ausgeben des Differenzsignals (D) und/oder eines Kontrollsignals eingerichtet ist, welche eine Abweichung zwischen dem Differenzsignal (D) und dem Referenzsignal (R) repräsentiert.

3. Medizinisches System (1a) nach einem der vorhergehenden Ansprüche, **dadurch gekennzeichnet, dass** wenigstens eine weitere, dritte Elektrode (13) an der Katheterspitze (5a) angeordnet und in proximaler Richtung von der zweiten Elektrode (7) beabstandet ist, wobei die dritte Elektrode (13) zum Erfassen eines dritten elektrischen Potentials (P3) und zum Erzeugen eines dritten Signals (S3) eingerichtet ist, welches einen Zeitverlauf des erfassten dritten elektrischen Potentials (P3) repräsentiert, wobei die Auswerteeinrichtung (3a) mit der dritten Elektrode (13) verbunden und eingerichtet ist zum Erzeugen eines weiteren Differenzsignals (d) in Abhängigkeit des zweiten Signals (S2) und des dritten Signals (S3), wobei das weitere Differenzsignal (d) einen Zeitverlauf einer elektrischen Spannung zwischen der zweiten Elektrode (7) und der dritten Elektrode (13) repräsentiert, und wobei die Auswerteeinrichtung (3a) eingerichtet ist zum Vergleichen des weiteren Differenzsignals (d) und des Differenzsignals (D).

## Claims

1. A medical system (1, 1a) for checking a position and an orientation of a catheter tip (5, 5a) in a patient's body, having
a catheter (2, 2a), in particular a central venous catheter, with the catheter tip (5, 5a) on which at least a first electrode (6) and a second electrode (7) are arranged, the second electrode (7) being spaced apart from the first electrode (6) in the proximal direction, wherein the first electrode (6) is configured to detect a first electrical potential (P1) and to generate a first signal (S1) which represents a time curve of the first electrical potential (P1), and wherein the second electrode (7) is configured to detect a second electrical potential (P2) and to generate a second signal (S2) which represents a time curve of the second electrical potential (P2),
an evaluation device (3, 3a) which is connected to the first electrode (6) and the second electrode (7), wherein the evaluation device (3, 3a) is configured to generate a differential signal (D) as a function of the first signal (S1) and the second signal (S2), wherein the differential signal (D) represents a time curve of an electrical voltage between the first electrode (6) and the second electrode (7), wherein the evaluation device (3, 3a) is configured to compare the differential signal (D) with a reference signal (R) which represents a desired position and orientation of the catheter tip (5, 5a), and wherein the evaluation device (3, 3a) is configured to detect and evaluate a change in sign of the differential signal (D), wherein the change in sign represents a change in a polarity of the first electrode (6) and the second electrode (7).

2. The medical system (1, 1a) as claimed in claim 1, **characterized by** an output device (4) which is connected to the evaluation device (3, 3a) and is configured to output the differential signal (D) and/or a check signal which represents a deviation between the differential signal (D) and the reference signal (R).

3. The medical system (1a) as claimed in one of the preceding claims, **characterized in that** at least one further, third electrode (13) is arranged on the catheter tip (5a) and is spaced apart from the second electrode (7) in the proximal direction, wherein the third electrode (13) is configured to detect a third electrical potential (P3) and to generate a third signal (S3) which represents a time curve of the detected third electrical potential (P3), wherein the evaluation device (3a) is connected to the third electrode (13) and is configured to generate a further differential signal (d) as a function of the second signal (S2) and the third signal (S3), the further differential signal (d) representing a time curve of an electrical voltage between the second electrode (7) and the third electrode (13), and wherein the evaluation device (3a) is configured to compare the further differential signal (d) and the differential signal (D).

## Revendications

1. Système médical (1, 1a) de vérification d'une position et d'une orientation d'une extrémité de cathéter (5, 5a) dans le corps d'un patient, comprenant :
un cathéter (2, 2a), en particulier un cathéter veineux central, comportant l'extrémité de cathéter (5, 5a), sur laquelle sont agencées au moins une première électrode (6) et une deuxième électrode (7), la deuxième électrode (7) étant espacée de la première électrode (6) en direction proximale, la première électrode (6) étant conçue pour détecter un premier potentiel électrique (P1) et pour générer un premier signal (S1) qui représente une évolution temporelle du premier potentiel électrique (P1), et la deuxième électrode (7) étant conçue pour détecter un deuxième potentiel électrique (P2) et pour générer un deuxième signal (S2) qui représente une évolution temporelle du deuxième potentiel électrique (P2),
un dispositif d'évaluation (3, 3a), qui est connecté à la première électrode (6) et à la deuxième électrode (7), le dispositif d'évaluation (3, 3a) étant conçu pour générer un signal de différence (D) en fonction du premier signal (S1) et du deuxième signal (S2), le signal de différence (D) représentant une évolution temporelle d'une tension électrique entre la première électrode (6) et la deuxième électrode (7), le dispositif d'évaluation (3, 3a) étant conçu pour comparer le signal de différence (D) à un signal de référence (R) qui représente une position et une orientation souhaitées de l'extrémité de cathéter (5, 5a), et le dispositif d'évaluation (3, 3a) étant conçu pour détecter et évaluer un changement de signe du signal de différence (D), le changement de signe représentant un changement d'une polarité de la première électrode (6) et de la deuxième électrode (7).

2. Système médical (1, 1a) selon la revendication 1, **caractérisé par** un dispositif de sortie (4) connecté au dispositif d'évaluation (3, 3a), lequel est conçu pour fournir en sortie le signal de différence (D) et/ou un signal de contrôle qui représente un écart entre le signal de différence (D) et le signal de référence (R).

3. Système médical (1a) selon l'une quelconque des revendications précédentes, **caractérisé en ce qu'**au moins une électrode supplémentaire, à savoir une troisième électrode (13), est agencée sur l'extrémité de cathéter (5a) et est espacée de la deuxième électrode (7) en direction proximale, la troisième électrode (13) étant conçue pour détecter un troisième potentiel électrique (P3) et pour générer un troisième signal (S3) qui représente une évolution temporelle du troisième potentiel électrique (P3) détecté, le dispositif d'évaluation (3a) étant connecté à la troisième électrode (13) et étant conçu pour générer un signal de différence supplémentaire (d) en fonction du deuxième signal (S2) et du troisième signal (S3), le signal de différence supplémentaire (d) représentant une évolution temporelle d'une tension électrique entre la deuxième électrode (7) et la troisième électrode (13), et le dispositif d'évaluation (3a) étant conçu pour comparer le signal de différence supplémentaire (d) et le signal de différence (D).
